Europäisches Patentamt

European Patent Office

Office européen des brevets

(11) Publication number: **0 162 575 B1**

# EUROPEAN PATENT SPECIFICATION

(45) Date of publication of patent specification: **04.09.91**  (51) Int. Cl.⁵: **C07K 7/06**, C07K 7/20, A61K 37/02

(21) Application number: **85302702.7**

(22) Date of filing: **17.04.85**

The file contains technical information submitted after the application was filed and nct included in this specification

(54) **GnRH Antagonists VII.**

(30) Priority: **21.05.84 US 612072**
**21.09.84 US 653867**
**31.01.85 US 696699**

(43) Date of publication of application:
**27.11.85 Bulletin 85/48**

(45) Publication of the grant of the patent:
**04.09.91 Bulletin 91/36**

(84) Designated Contracting States:
**AT BE CH DE FR GB IT LI LU NL SE**

(56) References cited:
**EP-A- 0 038 135          EP-A- 0 041 286**
**EP-A- 0 049 628          EP-A- 0 063 423**
**EP-A- 0 081 877          EP-A- 0 100 218**
**EP-A- 0 122 712          GB-A- 2 009 182**
**US-A- 3 933 782          US-A- 4 504 414**

**UNLISTED DRUGS, vol. 34, no. 8, August 1982, page 122-m**

**UNLISTED DRUGS, vol. 34, no. 10, October 1982, page 157-e**

(73) Proprietor: **THE SALK INSTITUTE FOR BIO-**

**LOGICAL STUDIES**
**10010 North Torrey Pines Road**
**La Jolla California 92038(US)**

(72) Inventor: **Rivier, Jean Eduard Frederic**
**9674 Blackgold Road**
**La Jolla California 92037(US)**
Inventor: **Vale, Wylie Walker, Jr.**
**1643 Valdez**
**La Jolla California 92037(US)**

(74) Representative: **Allard, Susan Joyce et al**
**BOULT, WADE & TENNANT, 27 Furnival**
**Street**
**London EC4A 1PQ(GB)**

## Description

The present invention relates to peptides which inhibit the release of gonadotropins by the pituitary gland in mammalians, including humans, and to methods of preventing ovulation and/or inhibiting the release of steroids. More particularly, the present invention is directed to peptides which inhibit gonadal function and the release of the steroidal hormones, progesterone and testosterone.

The pituitary gland is attached by a stalk to the region in the base of the brain known as the hypothalamus. In particular, follicle stimulating hormone (FSH) and luteinizing hormone (LH), sometimes referred to as gonadotropins or gonadotropic hormones, are released by the pituitary gland. These hormones, in combination, regulate the functioning of the gonads to produce testosterone in the testes and progesterone and estrogen in the ovaries, and they also regulate the production and maturation of gametes.

The release of a hormone by the anterior lobe of the pituitary gland usually requires a prior release of another class of hormones produced by the hypothalamus. One of the hypothalamic hormones acts as a factor that triggers the release of the gonadotropic hormones, particularly LH, and this hormone is referred to herein as GnRH although it has also been referred to as LH-RH and as LRF. GnRH has been isolated and characterized as a decapeptide having the following structure:

p-Glu-His-Trp-Ser-Tyr-Gly-Leu-Arg-Pro-Gly-$NH_2$

Peptides are compounds which contain two or more amino acids in which the carboxyl group of one acid is linked to the amino group of the other acid. The formula for GnRH, as represented above, is in accordance with conventional representation of peptides where the amino terminus appears to the left and the carboxyl terminus to the right. The position of the amino acid residue is identified by numbering the amino acid residues from left to right. In the case of GnRH, the hydroxyl portion of the carboxyl group of glycine has been replaced with an amino group($NH_2$). The abbreviations for the individual amino acid residues above are conventional and are based on the trivial name of the amino acid, e.g. p-Glu is pyroglutamic acid, His is histidine, Trp is tryptophan, Ser is serine, Tyr is tyrosine, Gly is glycine, Leu is leucine, Orn is ornithine, Arg is arginine, Pro is proline, Sar is sarcosine, Phe is phenylalanine and Ala is alanine. These amino acids together with valine, isoleucine, threonine, lysine, aspartic acid, asparagine, glutamine, cysteine, methionine, phenylalanine, and proline are generally considered to be the common, naturally occurring or protein-derived amino acids. Except for glycine, amino acids of the peptides of the invention are of the L-configuration unless noted otherwise.

There are reasons for desiring to prevent ovulation in female mammalians, and the administration of GnRH analogs that are antagonistic to the normal function of GnRH have been used to suppress or delay ovulation. For this reason, analogs of GnRH which are antagonistic to GnRH are being investigated for their potential use as a contraceptive or for regulating conception periods. GnRH antagonists may also be used for the treatment of precocious puberty and endometriosis. Such antagonists have also been found useful to regulate the secretion of gonadotropins in male mammals and can be employed to arrest spermatogenesis, e.g. as male contraceptives, and for treatment of prostatic hypertrophy. It is desired to provide improved peptides which are strongly antagonistic to endogenous GnRH and which prevent secretion of LH and the release of steroids by the gonads of mammals.

EP-A-0100218 discloses certain peptides of the structure.

X-$\beta$-D-2NAL-$R_2$-D-Trp-Ser-$R_5$-$R_6$-$R_7$-Arg-Pro-$R_{10}$ where $R_6$ is 4-$NH_2$-D-Phe or D-Arg.

These peptides inhibit the secretion of gonadotropins by the pituitary gland and inhibit the release of steroids by the gonads.

We have now developed certain improved peptides which have the amino acid residue D-3PAL or (Y)D-Trp, where Y is $NO_2$, $NH_2$, $OCH_3$, F, Cl, Br, $CH_3$, $N^{in}$For or $N^{in}$Ac at the third position in the peptide chain and amino acid residues other than 4$NH_2$-D-Phe or D-Arg at the sixth position in the peptide chain.

The present invention thus provides peptides which inhibit the release of gonadotropins in mammalians, including humans, and it also provides methods for inhibiting the release of steroids by the gonads of male and female mammalians. The improved GnRH analogs are strongly antagonistic to GnRH and have an inhibitory effect on the reproduction processes of mammalians. These analogs may be used to inhibit the production of gonadotropins and sex hormones under various circumstances including precocious puberty, hormone dependent neoplasia, dysmenorrhea and endometriosis.

Generally, in accordance with the present invention, peptides have been synthesized which strongly inhibit the secretion of gonadotropins by the pituitary gland of mammalians, including humans, and/or inhibit the release of steroids by the gonads.

Accordingly the present invention provides a peptide or a nontoxic salt thereof, said peptide having the formula:

X-$R_1$-(W)D-Phe-$R_3$-$R_4$-Arg-$R_6$-$R_7$-Arg-Pro-$R_{10}$

wherein X is hydrogen or an acyl group having 7 or less carbon atoms; $R_1$ is dehydro-Pro, or $\beta$-D-NAL (the D-isomer of alanine which is substituted by naphthyl on the $\beta$-carbon atom); W is Cl, F, $NO_2$, $C^\alpha Me/4Cl$, $Cl_2$ or Br; $R_3$ is D-3PAL (the D-isomer of alanine which is substituted by pyridyl on the $\beta$-carbon atom with the linkage to the 3-position of the pyridyl ring) or (Y)D-Trp, with Y being $NO_2$ or $N^{in}For$; $R_4$ is Ser, Orn, AAL ($\beta$-amino alanine) or aBu ($\gamma$-diamino butyric acid); $R_6$ is D-Trp, $NO_2$-D-Trp, D-Leu, D-Ile, D-Nle, D-Tyr, D-Val, D-Ala, D-Ser(OtBu), $\beta$-D-NAL, (imBzl)D-His or D-PAL (the D-isomer of alanine which is substituted by pyridyl on the $\beta$-carbon atom); $R_7$ is Nle, Leu, NML(N $CH_3$-L-Leu), Phe, Met, Nva, Tyr, Trp, Cys, PAL or 4F-D-Phe; and $R_{10}$ is Gly-$NH_2$, D-Ala-$NH_2$ or NH-Y', with Y' being lower alkyl, cycloalkyl, fluoro lower alkyl or NHCOHN-Q, where Q is H or lower alkyl.

The peptides of the present invention are analogs of GnRH wherein there is a 1-position substitution of dehydro-Pro or $\beta$-(1-or 2-naphthyl)-D-alanine (hereinafter $\beta$-D-1NAL or $\beta$-D-2NAL), a 2-position substitution in the form of a modified D-Phe, a 3-position substitution in the form of substituted D-Trp or D-3PAL, an optional substitution of a diamino acid having not more than 5 carbon atoms in the 4-position, a substitution in the 5-position of Arg, a 6-position substitution and an optional substitution in the 7-position such as Nle, NML, Phe, Nva, Met, Tyr, Try, Cys, PAL and 4F-D-Phe. Modified D-Phe in the 2-position provides increased antagonistic activity as a result of the specific modifications present in the benzene ring. Single substitutions for hydrogen in the ring are made in the para- or 4-position, and the substitutions are selected from chloro, fluoro, bromo and nitro, with chloro, fluoro and nitro being preferred. Dichloro substitutions are in the 2,4 or 3,4 positions in the ring. The alpha-carbon atom may also be methylated, e.g. C Me/4Cl)Phe. The 1-position substituent may be modified so that its alpha amino group contains an acyl group, such as formyl(For), acetyl, acrylyl, vinylacetyl(Vac) or benzoyl(Bz), with acetyl(Ac) and acrylyl(Acr) being preferred. PAL and D-PAL represent the L- and D-isomers of pyridyl-alanine where the $\beta$-carbon of Ala is linked, preferably, to the 3-position on the pyridine ring.

When dehydro-Pro is present in the 1-position, a D-isomer of a lipophilic amino acid, such as D-Trp, D-Phe, For-D-Trp, $NO_2$-D-Trp, D-Leu, D-Ile, D-Nle, D-Tyr, D-Val. D-Ala, D-Ser(OtBu), $\beta$-D-NAL or (imBzl)D-His is preferably in the 6-position, but D-PAL may be used. A substitution in the 10-position of D-Ala for Gly is considered optional, along with other substitutions mentioned hereinafter.

Because these peptides are highly potent to inhibit release of LH, they are often referred to as GnRH antagonists. The peptides inhibit ovulation of female mammals when administered at very low levels at proestrous and are also effective to cause resorption of fertilized eggs if administered shortly after conception. These peptides are also effective for the contraceptive treatment of male mammals.

By $\beta$-D-NAL is meant the D-isomer of alanine which is substituted by naphthyl on the $\beta$-carbon atom, i.e., also 3-D-NAL. preferably $\beta$-D-2NAL is employed, the attachment to naphthalene is at the 2-position on the ring structure; however, $\beta$-D-1NAL may also be used. PAL represents alanine which is substituted by pyridyl on the $\beta$-carbon atom; preferably the linkage is to the 3-position on the pyridine ring. In (Y) D-Trp in the 3-position, single substitutions for hydrogen are made in either the 5- or 6-position, with Y being selected from chloro, fluoro, bromo, methyl, amino, methoxy and nitro, with chloro, fluoro and nitro being preferred. Alternatively, the indole nitrogen may be acylated, e.g. with formyl ($N^{in}For$- or 1For-) or with acetyl. $N^{in}For$-D-Trp and $6NO_2$-D-Trp are the preferred substituted residues. By NML is meant $N^\alpha CH_3$-L-Leu. By AAL is meant $\beta$-amino-Ala and by aBu is meant $\alpha$, $\gamma$ diamino butyric acid, either of which or Orn can be present in the 4-position. When Ser is not present in the 4-position, dehydro Pro is preferably present in the 1-position. By 4-gua-D-Phe is meant a residue of D-Phe having guanidine substituted in the para-position.

The peptides of the present invention can be synthesized by classical solution synthesis or by a solid phase technique using a chloromethylated resin, a methylbenzhydrylamine resin (MBHA), a benzhydrylamine (BHA) resin or any other suitable resin known in the art. The solid phase synthesis is conducted in a manner to stepwise add the amino acids in the chain in the manner set forth in detail in the U.S. Patent No. 4,211,693. Side-chain protecting groups, as are well known in the art, are preferably added to Ser, Tyr and Arg when present, as well as to certain of the substituents, and may optionally be added to Trp, before these amino acids are coupled to the chain being built upon the resin. Such a method provides the fully protected intermediate peptidoresin.

The intermediates of the invention may be represented:

$X^1$-$R_1$-(W)D-Phe-$R_3$($X^2$)-$R_4$($X^3$)-Arg($X^4$ or $X^5$)-$R_6$($X^5$)-$R_7$($X^6$)-Arg($X^5$)-Pro-$X^7$ wherein: $X^1$ is an $\alpha$-amino protecting group of the type known to be useful in the art in the stepwise synthesis of polypeptides and when X in the desired peptide composition is a particular acyl group, that group may be used as the protecting group. Among the classes of $\alpha$-amino protecting groups covered by $X^1$ are (1) acyl-type protecting groups, such as formyl(For), trifluoroacetyl, phthalyl, p-toluenesulfonyl(Tos), benzoyl(Bz), benzenesulfonyl, o-nitrophenylsulfenyl(Nps), tritylsulfenyl, o-nitrophenoxyacetyl, acrylyl(Acr), chloroacetyl,

acetyl(Ac) and $\gamma$-chlorobutyryl; (2) aromatic urethan-type protecting groups, e.g., benzyloxycarbonyl (Z) and substituted benzyloxycarbonyl, such as p-chloro-benzyloxycarbonyl(ClZ), p-nitrobenzyloxycarbonyl, p-bromobenzyloxycarbonyl and p-methoxybenzyloxycarbonyl; (3) aliphatic urethan protecting groups, such as tertbutyloxycarbonyl(Boc), diisopropylmethoxycarbonyl, isopropyloxycarbonyl, ethoxycarbonyl and allyloxycarbonyl; (4) cycloalkyl urethan-type protecting groups, such as cyclopentyloxycarbonyl, adamantyloxycarbonyl and cyclohexyloxycarbonyl; (5) thiourethan-type protecting groups, such as phenylthiocarbonyl; (6) alkyl-type protecting groups, such as allyl(Aly), triphenylmethyl(trityl) and benzyl(Bzl); (7) trialkylsilane groups, such as trimethylsilane. The preferred $\alpha$-amino protecting group is Boc when X is hydrogen.

$X^2$ is hydrogen or a protecting group for the indole nitrogen of Trp, such as benzyl; however in many syntheses there is no need to protect Trp.

$X^3$ is hydrogen or a protecting group for the alcoholic hydroxyl group of Ser, such as one selected from the group consisting of acetyl, benzoyl, tetrahydropyranyl, tert-butyl, trityl, benzyl and 2,6-dichlorobenzyl, with benzyl being preferred. Alternatively, when a substitution is made for Ser, $X^3$ may be a protecting group for a side chain amino group, such as Tos, Z or ClZ.

$X^4$ is hydrogen or a protecting group for the phenolic hydroxyl group of Tyr, if Tyr is present, selected from the group consisting of tetrahydropyranyl, tert-butyl, trityl, benzyl, Z, 4-bromobenzyloxycarbonyl and 2,6-dichlorobenzyl. 2,6-dichlorobenzyl (DCB) is preferred.

$X^5$ is a protecting group for Arg such as nitro, Tos, trityl, benzyloxycarbonyl, adamantyloxycarbonyl, Z and Boc, Tos is generally preferred.

$X^6$ is hydrogen, a protecting group for Tyr, such as $X^4$ or a protecting group for Cys preferably selected from the class consisting of p-methoxybenzyl(MeOBzl), p-methylbenzyl, acetamidomethyl, trityl and Bzl. The most preferred protecting group is p-methoxybenzyl.

$X^7$ may be Gly-O-CH$_2$-[resin support]; O-CH$_2$-[resin support]; D-Ala-O-CH$_2$-[resin support]; Gly-NH-[resin support] or D-Ala-NH-[resin support]; and it may be OH, ester, amide or hydrazide either of Gly or D-Ala or attached directly to Pro.

The criterion for selecting side chain protecting groups for $X^2$-$X^6$ is that the protecting group should be stable to the reagent under the reaction conditions selected for removing the $\alpha$-amino protecting group at each step of the synthesis. The protecting group should not be split off under coupling conditions, and the protecting group should be removable upon completion of the synthesis of the desired amino acid sequence under reaction conditions that will not alter the peptide chain.

When the $X^7$ group is Gly-O-CH$_2$-[resin support], D-Ala-O-CH$_2$-[resin support] or O-CH$_2$-[resin support], the ester moiety of one of the many functional groups of the polystyrene resin support is being represented. When the $X^7$ group is Gly-NH-[resin support] or D-Ala-NH-[resin support], an amide bond connects Gly or D-Ala to BHA resin or to a MBHA resin.

When X is acetyl, for example, in the final formula, it may be possible to employ it as the $X^1$ protecting group for the $\alpha$-amino group of D-NAL or whatever amino acid is used in the 1-position by adding it before the coupling of this last amino acid to the peptide chain. However, a reaction is preferably carried out with the peptide on the resin (after deblocking the $\alpha$-amino group while the side-chain groups remain protected), e.g. by reacting with acetic acid in the presence of dicyclohexyl carbodiimide (DCC) or preferably with acetic anhydride or by another suitable reaction as known in the art.

The fully protected peptide can be cleaved from a chloromethylated resin support by ammonolysis, as is well known in the art, to yield the fully protected amide intermediate. Deprotection of the peptide, as well as cleavage of the peptide from a benzhydrylamine resin, can take place at $0\,^{\circ}C$ with hydrofluoric acid (HF). Anisole is preferably added to the peptide prior to treatment with HF. After the removal of HF, under vacuum, the cleaved, deprotected peptide is conveniently treated with ether, decanted, taken-up in dilute acetic acid and lyophilized.

Thus the invention also provides a method for making a peptide or a nontoxic salt thereof, said peptide having the formula:

X-R$_1$-(W)D-Phe-R$_3$-R$_4$-Arg-R$_6$-R$_7$-Arg-Pro-R$_{10}$

wherein X is hydrogen or an acyl group having 7 or less carbon atoms; R$_1$ is dehydro-Pro, or $\beta$-D-NAL (the D-isomer of alanine which is substituted by naphthyl on the $\beta$-carbon atom); W is Cl, F, NO$_2$, C$^\alpha$Me/4Cl, Cl$_2$ or Br; R$_3$ is D-3PAL (the D-isomer of alanine which is substituted by pyridyl on the $\beta$-carbon atom with the linkage to the 3-position of the pyridyl ring) or (Y)D-Trp, with Y being NO$_2$ or N$^{in}$For; R$_4$ is Ser, Orn, AAL ($\beta$-amino alanine) or aBu ($\gamma$-diamino butyric acid); R$_6$ is D-Trp, NO$_2$-D-Trp, D-Leu, D-Ile, D-Nle, D-Tyr, D-Val, D-Ala, D-Ser(OtBu), $\beta$-D-NAL, (imBzl)D-His or D-PAL (the D-isomer of alanine which is substituted by pyridyl on the $\beta$-carbon atom); R$_7$ is Nle, Leu, NML(N CH$_3$-L-Leu), Phe, Met, Nva, Tyr, Trp, Cys, PAL or 4F-D-Phe; and R$_{10}$ is Gly-NH$_2$, D-Ala-NH$_2$ or NH-Y', with Y' being lower alkyl, cycloalkyl, fluoro lower alkyl or NHCOHN-Q, where Q is H or lower alkyl, which method comprises (a) forming an intermediate compound

4

having the formula:

1-$R_1$-(w)D-Phe-$R_3$($X^2$)-$R_4$($X^3$)-Arg($X^4$ or $X^5$)-$R_6$($X^5$)-$R_7$($X^6$)-Arg($X^5$)-Pro-$X^7$, wherein $X^1$ is hydrogen or an α-amino protecting group; $X^2$ is hydrogen or a protecting group for the indole nitrogen; $X^3$ is hydrogen or a protecting group for the alcoholic hydroxyl group of Ser or for a side-chain amino group; $X^4$ is hydrogen or a protecting group for the phenolic hydroxyl group of Tyr; $X^5$ and $X^6$ are each either hydrogen or a protecting group for the respective side chain that is present; and $X^7$ is selected from the group consisting of Gly-O-$CH_2$-(resin support), O-$CH_2$-(resin support), D-Ala-O-$CH_2$-(resin support), Gly-NH-(resin support), D-Ala-NH-(resin support), Gly-$NH_2$, and esters, amides and hydrazides; (b) splitting off one or more of the groups $X^1$ to $X^6$ and/or cleaving from any resin support included in $X^7$ and, if desired, (c) converting a resulting peptide into a nontoxic salt thereof.

Purification of the peptide is effected by ion exchange chromotography on a CMC column, followed by partition chromatography using the elution system: n-butanol;0.1N acetic acid (1:1 volume ratio) on a column packed with Sephadex G-25, or by using HPLC, as known in the art and specifically set forth in J. Rivier, et al. J. Chromatography, 288 (1984) 303-328.

The peptides of the invention are effective at levels of less than 100 micrograms per kilogram of body weight, when administered at about noon on the day of proestrous, to prevent ovulation in female rats. For prolonged suppression of ovulation, it may be necessary to use dosage levels in the range of from about 0.1 to about 2.5 milligrams per kilogram of body weight. These antagonists are also effective to arrest spermatogenesis when administered to male mammals on a regular basis and can thus be used as contraceptives. Since these compounds will reduce testosterone levels (an undesired consequence in the normal, sexually active male), it may be reasonable to administer replacement dosages of testosterone along with the GnRH antagonist. These antagonists can also be used to regulate the production of gonadotropins and sex steroids for other purposes as indicated hereinbefore.

## EXAMPLE I

Peptides as indicated in TABLE I having the formula:

Ac-$R_1$-(4F)D-Phe-$R_3$-Ser-Tyr-$R_6$-Leu-Arg-Pro-$R_{10}$

are prepared by the solid-phase procedure referred to above. The peptides listed in Table I are not Examples of the invention.

### TABLE I

|  | $R_1$ | $R_3$ | $R_6$ | $R_{10}$ |
|---|---|---|---|---|
| 1 | β-D-2NAL | ($6NO_2$)D-Trp | D-Arg | Gly-$NH_2$ |
| 3 | dehydro Pro | • | β-D-2NAL | Gly-$NH_2$ |
| 13 | dehydro Pro | ($6NO_2$)D-Trp | D-Leu | $NHCH_2CH_3$ |
| 14 | D-Trp | (5F)D-Trp | D-Phe | • |
| 15 | D-pGlu | (5F)D-Trp | D-Ile | D-Ala-$NH_2$ |
| 16 | D-Phe | ($6NO_2$)D-Trp | D-Val | $NHNHCONH_2$ |

For purposes of an example, a representative solid phase synthesis of Peptide No. 1 above, which is referred to as [Ac-β-D-2NAL[1], (4F)D-Phe[2], ($6NO_2$)D-Trp[3], D-Arg[6]]-GnRH is set forth hereinafter. This peptide has the following formula: Ac-β-D-2NAL-(4F)D-Phe-($6NO_2$)D-Trp-Ser-Tyr-D-Arg-Leu-Arg-Pro-Gly-$NH_2$. The other peptides are similarly synthesized and purified.

A BHA resin is used, and Boc-protected Gly is coupled to the resin over a 2-hour period in $CH_2Cl_2$ using a 3-fold excess of Boc derivative and DCC as an activating reagent. The glycine residue attaches to the BHA residue by an amide bond.

Following the coupling of each amino acid residue, washing, deblocking and coupling of the next amino acid residue is carried out in accordance with the following schedule using an automated machine and beginning with about 5 grams of resin:

| STEP | REAGENTS AND OPERATIONS | MIX TIMES MIN. |
|---|---|---|
| 1 | $CH_2Cl_2$ wash-80 ml. (2 times) | 3 |
| 2 | Methanol(MeOH) wash-30 ml. (2 times) | 3 |
| 3 | $CH_2Cl_2$ wash-80 ml. (3 times) | 3 |
| 4 | 50 percent TFA plus 5 percent 1,2-ethanedithiol in $CH_2Cl_2$-70 ml. (2 times) | 10 |
| 5 | Isopropyl alcohol + 1% ethanedithiol wash-80 ml. (2 times) | 3 |
| 6 | TEA 12.5 percent in $CH_2Cl_2$-70 ml. (2 times) | 5 |
| 7 | MeOH wash-40 ml. (2 times) | 2 |
| 8 | $CH_2Cl_2$ wash-80 ml. (3 times) | 3 |
| 9 | Boc-amino acid (10 mmoles) in 30 ml. of either DMF or $CH_2Cl_2$, depending upon the solubility of the particular protected amino acid, (1 time) plus DCC (10 mmoles) in $CH_2Cl_2$ | 30-300 |
| 10 | MeOH wash-40 ml. (2 times) | 3 |
| 11 | TEA 12.5 percent in $CH_2Cl_2$-70 ml. (1 time) | 3 |
| 12 | MeOH wash-30 ml. (2 times) | 3 |
| 13 | $CH_2Cl_2$ wash-80 ml. (2 times) | 3 |

After step 13, an aliquot may be taken for a ninhydrin test: if the test is negative, go back to step 1 for coupling of the next amino acid; if the test is positive or slightly positive, go back and repeat steps 9 through 13.

The above schedule is used for coupling of each of the amino acids of the peptide of the invention after the first amino acid has been attached. NαBoc protection is used for each of the remaining amino acids throughout the synthesis. NαBoc-β-D-2NAL is prepared by a method known in the art, e.g. as described in detail in U.S. Patent No. 4,234,571, issued November 18, 1980. The side chain of Arg is protected with Tos. OBzl is used as a side chain protecting group for the hydroxyl group of Ser. $(6NO_2)$D-Trp is left unprotected. NαBoc-β-D-2NAL is introduced as the final amino acid. Boc-Arg(Tos) and Boc-$(6NO_2)$D-Trp, which have low solubility in $CH_2Cl_2$, are coupled using DMF:$CH_2Cl_2$ mixtures.

After deblocking the α-amino group at the N-terminal, acetylation is achieved using a large excess of acetic anhydride in dichloromethane. The cleavage of the peptide from the resin and complete deprotection of the side chains takes place very readily at 0° C. with HF. Anisole is added as a scavenger prior to HF treatment. After the removal of HF under vacuum, the resin is extracted with 50% acetic acid, and the washings are lyophilized to provide a crude peptide powder.

Purification of the peptide is then effected by ion exchange chromatography on CMC (Whatman CM 32, using a gradient of 6.05 to 0.3M $NH_4OAc$ in 50/50 methanol/water) followed by partition chromatography in a gel filtration column using the elution system: n-Butanol; 0.1N Acetic acid (1:1 - volume ratio).

The peptide is judged to be homogeneous using thin layer chromatography and several different solvent systems, as well as by using reversed-phase high pressure liquid chromatography and an aqueous triethylammonium phosphate solution plus acetonitrile. Amino acid analysis of the resultant, purified peptide is consistent with the formula for the prepared structure, showing substantially integer-values for each amino acid in the chain. The optical rotation is measured on a photoelectric polarimeter as $[\alpha]_D^{22}$ = -31.8° ±1(c = 1, 50% acetic acid).

The peptides are assayed in vivo and may also be tested in vitro. If performed, in vitro testing is carried out using dissociated rat pituitary cells maintained in culture for 4 days prior to the assay. The levels of LH mediated in response to the application of peptides is assayed by specific radioimmunoassay for rat LH.

Control dishes of cells only receive a measure which is 3 nanomolar in GnRH; experimental dishes receive a measure 3 nanomolar in GnRH plus a measure having either the present standard antagonist for comparison purposes i.e. [Ac-dehydro Pro$^1$, (4-F)D-Phe$^2$, D-Trp$^{3,6}$]-GnRH or the test peptide, in concentrations ranging from 0.01 to 10 nanomolar. The amount of LH secreted in the samples treated only with GnRH is compared with that secreted by the samples treated with the peptide plus GnRH. The ability of the test peptide to reduce the amount of LH released by 3 nanomolar GnRH is compared to that of the present standard peptide. The in vivo testing determines effectiveness to prevent ovulation in female rats. In this test, a specified number of mature female Sprague-Dawley rats, i.e. six, each having a body weight from 225 to 250 grams, is injected with a specified microgram dosage of peptide in corn oil at about noon on the day of proestrous. Proestrous is the afternoon of ovulation. A separate female rat group is used as a control to which the peptide is not administered. Each of the control female rats ovulates on the evening of proestrous; of the rats treated, the number of them which ovulate is recorded. Each of the peptides is considered to be significantly effective to prevent ovulation of female rats at a very low dosage, and each peptide is considered to be totally effective at a dose of about five micrograms. Additional testing is carried out at lower dosages with the results being set forth in TABLE A hereinafter.

## TABLE A

| Peptide No. | in vivo Dose (µg) | No. Ovulating |
|---|---|---|
| 3. | 2.5 | 2/10 |
| | 2 | 0/6 |

All peptides listed in Table I are considered effective to block GnRH-induced LH secretion in vitro at some reasonable concentration. Many of these peptides are much more potent in vivo than the present standard. All of the peptides are considered to be effective to prevent ovulation of female mammals at very low dosages, and some selected ones are considered to be at least twice as potent as any GnRH antagonists previously known and tested.

## EXAMPLE II

Peptides as indicated in TABLE II having the formula:
X-$\beta$-D-2NAL-(4Cl)D-Phe-(6NO$_2$)D-Trp-Ser-R$_5$-R$_6$-NML-Arg-Pro-R$_{10}$ are prepared by the solid-phase procedure referred to above.

## TABLE II

| | X | R$_5$ | R$_6$ | R$_{10}$ |
|---|---|---|---|---|
| 33 | Acr | Arg | D-Tyr | D-Ala-NH$_2$ |
| 34 | For | " | " | NHCH$_2$CH$_3$ |

In vitro and/or in vivo testing of the peptides specified in Table II shows that the peptides listed in Table II are considered effective to block GnRH-induced LH secretion in vitro at a reasonable concentration. Many of these peptides are more potent in vivo than the present standard. All of the peptides are considered to be effective to prevent ovulation of female mammals at very low dosages.

## EXAMPLE III

Peptides as indicated in TABLE III having the formula: Ac-R$_1$-R$_2$-D-3PAL-Ser-Arg-R$_6$-R$_7$-Arg-Pro-R$_{10}$ are prepared by the solid phase procedure referred to above.

7

## TABLE

| | | $R_1$ | $R_2$ | $R_6$ | $R_7$ | $R_{10}$ |
|---|---|---|---|---|---|---|
| 101 | | ß-D-2NAL | 4Cl-D-Phe | D-Trp | Leu | D-Ala-NH$_2$ |
| 102 | | " | " | D-3PAL | " | " |
| 103 | | " | " | ß-D-2NAL | " | " |
| 104 | | " | " | D-3PAL | " | " (4guaPhe$^5$. |
| 105 | | dehydroPro | " | ß-D-2NAL | 3PAL | D-Ala-NH$_2$ |
| 106 | | " | " | " | Tyr | " |
| 107 | | ß-D-2NAL | " | D-3PAL | NML | " |
| 108 | | " | " | ß-D-2NAL | 3PAL | " |
| 109 | | " | " | (imBzl)D-His | Leu | " |
| 110 | | " | " | 6NO$_2$-D-Trp | " | " |
| 111 | | " | " | D-Tyr | " | " |
| 112 | | " | " | (For)D-Trp | " | " |
| 113 | | " | 4NO$_2$-D-Phe | D-Trp | NML | " |
| 114 | | " | 4Br-D-Phe | D-Tyr | Nle | NHCH$_2$CH$_3$ |
| 115 | | Pro | " | (imBzl)D-His | Met | " |
| 116 | | dehydroPro | " | D-Trp | Nva | NHNHCONH$_2$ |
| 117 | | " | C$^\alpha$Me4Cl-D-Phe | " | " | NHCH$_2$CH$_3$ |
| 118 | | " | 4F-D-Phe | " | 4F-Phe | D-Ala-NH$_2$ |
| 119 | | " | " | " | NML | NHNHCONH$_2$ |
| 120 | | " | " | D-Trp | Nle | NHCH$_2$CH$_3$ |
| 121 | | " | " | ß-D-2NAL | Trp | Gly-NH$_2$ |
| 122 | | Pro | " | " | Nva | " |
| 123 | | ß-D-2NAL | 3,4Cl-D-Phe | ß-D-1NAL | Tyr | D-Ala-NH$_2$ |
| 124 | | " | " | D-Trp | Met | D-Ala-NH$_2$ |
| 125 | | 4Cl-D-Phe | " | D-Tyr | 3PAL | " (acetat salt) |

The peptides described in TABLE III are tested in vivo to determine their effectiveness to prevent ovulation in female rats. All of them are considered to prevent ovulation of female rats at a very low dosage, and to be totally effective at a dose of about ten micrograms. Specific testing of a number of these peptides was also carried out at lower dosages, with the results being set forth in TABLE C hereinafter.

## TABLE C

| Peptide No. | $[\alpha]_D^{22}$ | in vivo Dose (ug) | No. Ovulating |
|---|---|---|---|
| 101. | −30.3°±1 | 1 | 0/6 |
| | | 0.5 | 2/10 |
| 102. | | 1 | 1/16 |
| | | 0.5 | 5/10 |
| 103. | | 1 | 0/5 |
| | | 0.5 | 6/10 |
| | | 0.25 | 7/10 |
| 108. | | 1 | 9/10 |
| 109. | | 0.5 | 0/6 |
| 110. | | 0.5 | 0/5 |

The peptides of the invention are often administered in the form of pharmaceutically acceptable, nontoxic salts, such as acid addition salts, or of metal complexes, e.g., with zinc, barium, calcium, magnesium, aluminum or the like (which are considered as addition salts for purposes of this application), or of combinations of the two. Illustrative of such acid addition salts are hydrochloride, hydrobromide, sulphate, phosphate, nitrate, oxalate, fumarate, gluconate, tannate, maleate, acetate, citrate, benzoate, succinate, alginate, malate, ascorbate, tartrate and the like. For example, an aqueous solution of the peptide can be repeatedly treated with 1N acetic acid and then lyophilized to yield the acetic acid salt thereof. If the active ingredient is to be administered in tablet form, the tablet may contain a pharmaceutically-acceptable diluent which includes a binder, such as tragacanth, corn starch or gelatin; a disintegrating agent, such as alginic acid; and a lubricant, such as magnesium stearate. If administration in liquid form is desired, sweetening and/or flavoring may be used as part of the pharmaceutically-acceptable diluent, and intravenous administration in isotonic saline, phosphate buffer solutions or the like may be effected.

The pharmaceutical compositions will usually contain the peptide in conjunction with a conventional, pharmaceutically-acceptable carrier. Usually, the dosage will be from about 1 to about 100 micrograms of the peptide per kilogram of the body weight of the host when given intravenously; oral dosages will be higher. Overall, treatment of subjects with these peptides is generally carried out in the same manner as the clinical treatment using other antagonists of GnRH.

These peptides can be administered to mammals intravenously, subcutaneously, intramuscularly, orally, percutaneously, e.g. intranasally or intravaginally to achieve fertility inhibition and/or control and also in applications calling for reversible suppression of gonadal activity, such as for the management of precocious puberty or during radiation-or chemo-therapy. Effective dosages will vary with the form of administration and the particular species of mammal being treated. An example of one typical dosage form is a bacteriostatic water solution containing the peptide which solution is administered to provide a dose in the range of about 0.1 to 2.5 mg/kg of body weight. Oral administration of the peptide may be given in either solid form or liquid form.

Although the invention has been described with regard to its preferred embodiments, it should be understood that changes and modifications as would be obvious to one having the ordinary skill in this art may be made without departing from the scope of the invention which is set forth in the claims which are appended hereto. For example, other substitutions known in the art which do not significantly detract from the effectiveness of the peptides may be employed in the peptides of the invention. The substitutions in the phenyl ring of the D-Phe[2] residue may also be in the 3-position and in the 2,4 positions, which are considered equivalents; similarly, D-2PAL and D-4PAL are considered to be equivalents of D-3PAL. At the C-terminus, Pro[9] can be linked to one of the following moieties which are considered to generally be equivalents thereof: Gly-OCH₃, Gly-OCH₃CH₃,Sar-NH₂, or NH-Y', with Y' being lower alkyl, particularly ethyl, cycloalkyl, fluoro lower alkyl or NHCONHQ, where Q is H or lower alkyl. Sar stands for sarcosine.

## Claims

**Claims for the following Contracting States:BE, CH, DE, FR, GB, IT, LI, LU, NL, SE.**

1. A peptide or a nontoxic salt thereof, said peptide having the formula:

X-$R_1$-(W)D-Phe-$R_3$-$R_4$-Arg-$R_6$-$R_7$-Arg-Pro-$R_{10}$

wherein X is hydrogen or an acyl group having 7 or less carbon atoms; $R_1$ is dehydro-Pro, or $\beta$-D-NAL (the D-isomer of alanine which is substituted by naphthyl on the $\beta$-carbon atom); W is Cl, F, $NO_2$, $C^\alpha$Me/4Cl, $Cl_2$ or Br; $R_3$ is D-3PAL (the D-isomer of alanine which is substituted by pyridyl on the $\beta$-carbon atom with the linkage to the 3-position of the pyridyl ring) or (Y)D-Trp, with Y being $NO_2$ or $N^{in}$For; $R_4$ is Ser, Orn, AAL ($\beta$-amino alanine) or aBu ($\gamma$-diamino butyric acid); $R_6$ is D-Trp, $NO_2$-D-Trp, D-Leu, D-Ile, D-Nle, D-Tyr, D-Val, D-Ala, D-Ser(OtBu), $\beta$-D-NAL, (imBzl)D-His or D-PAL (the D-isomer of alanine which is substituted by pyridyl on the $\beta$-carbon atom); $R_7$ is Nle, Leu, NML($N^\alpha$CH$_3$-L-Leu), Phe, Met, Nva, Tyr, Trp, Cys, PAL or 4F-D-Phe; and $R_{10}$ is Gly-$NH_2$, D-Ala-$NH_2$ or NH-Y', with Y' being lower alkyl, cycloalkyl, fluoro lower alkyl or NHCOHN-Q, where Q is H or lower alkyl.

2. A peptide as claimed in claim 1 wherein W is 4Cl of 4F.

3. A peptide as claimed in claim 1 or claim 2 wherein $R_1$ is $\beta$-D-2NAL and $R_6$ is D-PAL.

4. A peptide as claimed in claim 2 wherein $R_6$ is (imBzl)D-His.

5. A peptide as claimed in any one of claims 1 to 4 wherein $R_7$ is Tyr or 3PAL.

6. A peptide as claimed in any one of claims 1 to 5 wherein $R_3$ is (Y)D-Trp.

7. A peptide as claimed in claim 1 or claim 2 wherein $R_1$ is $\beta$-D-NAL and $R_3$ is D-PAL and $R_5$ is Arg.

8. A peptide as claimed in claim 7 wherein $R_6$ is (imBzl) D-His or ($6NO_2$) D = Trp and $R_7$ is Leu.

9. A peptide as claimed in any one of claims 1 to 7 wherein $R_4$ is Ser, X is Ac or Acr and $R_{10}$ is D-Ala-$NH_2$, or Gly-$NH_2$.

10. A pharmaceutical composition for regulating the secretion of gonadotropins in mammals which comprises as an active ingredient an effective amount of a peptide as claimed in any one of claims 1 to 8, together with a pharmaceutically acceptable diluent or carrier.

## Claims for the following Contracting State: AT

1. A method for making a peptide or a nontoxic salt thereof having the formula:

X-$R_1$-(W)D-Phe-$R_3$-$R_4$-Arg-$R_6$-$R_7$-Arg-Pro-$R_{10}$

wherein X is hydrogen or an acyl group having 7 or less carbon atoms; $R_1$ is dehydro-Pro, or $\beta$-D-NAL (the D-isomer of alanine which is substituted by naphthyl on the $\beta$-carbon atom); W is Cl, F, $NO_2$, $C^\alpha$Me/4Cl, $Cl_2$ or Br; $R_3$ is D-3PAL (the D-isomer of alanine which is substituted by pyridyl on the $\beta$-carbon atom with the linkage to the 3-position of the pyridyl ring) or (Y)D-Trp, with Y being $NO_2$ or $N^{in}$For; $R_4$ is Ser, Orn, AAL ($\beta$-amino alanine) or aBu ($\gamma$-diamino butyric acid); $R_6$ is D-Trp, $\beta$-D-NAL, (imBzl)D-His or D-PAL (the D-isomer of alanine which is substituted by pyridyl on the $\beta$-carbon atom); $R_7$ is Nle, Leu, NML($N^\alpha$CH$_3$-L-Leu), Phe, Met, Nva, Tyr, Trp, Cys, PAL or 4F-D-Phe; and $R_{10}$ is Gly-$NH_2$, D-Ala-$NH_2$ or NH-Y', with Y' being lower alkyl, cycloalkyl, fluoro lower alkyl or NHCOHN-Q, where Q is H or lower alkyl; which method comprises (a) forming an intermediate compound having the formula: $X^1$-$R_1$-(W)D-Phe-$R_3$($X^2$)-$R_4$($X^3$)Arg($X^5$)-$R_6$($X^5$)-$R_7$($X^6$)-Arg($X^5$)-Pro-$X^7$, wherein $X^1$ is hydrogen or an $\alpha$-amino protecting group; $X^2$ is hydrogen or a protecting group for the indole nitrogen; $X^3$ is hydrogen or a protecting group for the alcoholic hydroxyl group of Ser or for a side-chain amino group; $X^5$ and $X^6$ are each either hydrogen or a protecting group for the respective side chain that is present; and $X^7$ is selected from the group consisting of Gly-O-$CH_2$-(resin support), O-$CH_2$-resin support), D-Ala-O-$CH_2$-(resin support), Gly-NH-(resin support), D-Ala-NH-(resin support), Gly-$NH_2$, and esters, amides and hydrazides; (b) splitting off one or more of the groups $X^1$ to $X^6$ and/or cleaving from any resin support included in $X^7$ and, if desired, (c) converting a resulting peptide into a nontoxic salt thereof.

2. A method as claimed in claim 1 wherein W is 4Cl of 4F.

3. A method as claimed in claim 1 or claim 2 wherein $R_1$ is $\beta$-D-2NAL and $R_6$ is D-PAL.

4. A method as claimed in claim 2 wherein $R_6$ is (imBzl)D-His.

5. A method as claimed in any one of claims 1 to 4 wherein $R_7$ is Tyr or 3PAL.

6. A method as claimed in any one of claims 1 to 5 wherein $R_3$ is (Y)D-Trp.

7. A method as claimed in claim 1 or claim 2 wherein $R_1$ is $\beta$-D-NAL and $R_3$ is D-PAL.

8. A method as claimed in claim 7 wherein $R_6$ is (imBZl)D-His or (6NO$_2$)D-Trp and $R_7$ is Leu.

9. A method as claimed in any one of claims 1 to 8 wherein $R_4$ is Ser, X is Ac or Acr and $R_{10}$ is D-Ala-NH$_2$ or Gly-NH$_2$.

10. A pharmaceutical composition for regulating the secretion of gonadotropins in mammals which comprises as an active ingredient an effective amount of a peptide as defined in any one of claims 1 to 9, together with a pharmaceutically acceptable diluent or carrier.

**Revendications**
**Revendications pour les Etats contractants suivants: BE, CH, DE, FR, GB, IT, LI, LU, NL, SE.**

1. Peptide ou sel atoxique de celui-ci, ledit peptide répondant à la formule :
X-$R_1$-(W)D-Phe-$R_3$-$R_4$-Arg-$R_6$-$R_7$-Arg-Pro-$R_{10}$,
dans laquelle X représente un atome d'hydrogène ou un groupe acyle ayant 7 atomes de carbone ou moins : $R_1$ représente déhydro-Pro ou $\beta$-D-NAL (l'isomère D d'alanine qui a été substituée par le napthyle sur l'atome de carbone $\beta$) ; W représente Cl, F, NO$_2$, C$^\alpha$Me/4Cl, Cl$_2$ ou Br ; $R_3$ représente D-3PAL (l'isomère D d'alanine qui a été substituée par le pyridyle sur l'atome de carbone $\beta$ avec la liaison en position 3 du cycle de pyridyle) ou (Y)D-Trp, Y représentant NO$_2$ ou N$^{in}$For ; $R_4$ représente Ser, Orn, AAL ($\beta$-amino-alanine) ou aBu (acide $\gamma$-diaminobutyrique) ; $R_6$ représente D-Trp, NO$_2$-D-Trp, D-Leu, D-Ile, D-Nle, D-Tyr, D-Val, D-Ala, D-Ser(OtBu), $\beta$-D-NAL, (imBzl)D-His ou D-PAL (l'isomère D d'alanine qui a été substituée par le pyridyle sur l'atome de carbone $\beta$) ; $R_7$ représente Nle, Leu, NML-(N CH$_3$-L-Leu), Phe, Met, Nva, Tyr, Trp, Cys, PAL ou 4F-D-Phe : et $R_{10}$ représente Gly-NH$_2$, D-Ala-NH$_2$ ou NH-Y', Y' représentant un alcoyle inférieur, un cycloalcoyle, un fluoro-alcoyle inférieur ou NHCOHN-Q, Q représentant H ou un alcoyle inférieur.

2. Peptide selon la revendication 1, dans lequel W représente 4Cl ou 4F.

3. Peptide selon la revendication 1 ou 2, dans lequel $R_1$ représente $\beta$-D-2NAL et $R_6$ représente D-PAL.

4. Peptide selon la revendication 2, dans lequel $R_6$ représente (imBzl)D-His.

5. Peptide selon l'une quelconque des revendications 1 à 4, dans lequel $R_7$ représente Tyr ou 3PAL.

6. Peptide selon l'une quelconque des revendications 1 à 5, dans lequel $R_3$ représente (Y)D-Trp.

7. Peptide selon la revendication 1 ou 2, dans lequel $R_1$ représente $\beta$-D-NAL et $R_3$ représente D-PAL et $R_5$ représente Arg.

8. Peptide selon la revendication 7, dans lequel $R_6$ représente (imBzl)D-His ou (6NO$_2$)D-Trp et $R_7$ représente Leu.

9. Peptide selon l'une quelconque des revendications 1 à 7, dans lequel $R_4$ représente Ser, X représente Ac ou Acr et $R_{10}$ représente D-Ala-NH$_2$ ou Gly-NH$_2$.

10. Composition pharmaceutique pour réguler la sécrétion de gonadotrophines chez les mammifères,

EP 0 162 575 B1

caractérisée en ce qu'elle comprend comme ingrédient actif une quantité efficace d'un peptide selon l'une quelconque des revendications 1 à 9, conjointement avec un diluant ou excipient pharmaceutiquement acceptable.

**Revendications pour l'Etat contractant suivant: AT.**

1. Procédé de préparation d un peptide ou d'un sel atoxique de celui-ci, répondant à la formule :
X-$R_1$ -(W)D-Phe-$R_3$-$R_4$-Arg-$R_6$-$R_7$-Arg-Pro-$R_{10}$,
dans laquelle X représente un atome d'hydrogène ou un groupe acyle ayant 7 atomes de carbone ou moins ; $R_1$ représente déhydro-Pro ou $\beta$-D-NAL (l'isomère D d'alanine qui a été substituée par le napthyle sur l'atome de carbone $\beta$) ; W représente Cl, F, $NO_2$, $C^\alpha Me/4Cl$, $Cl_2$ ou Br ; $R_3$ représente D-3PAL (l'isomère D d'alanine qui a été substituée par le pyridyle sur l'atome de carbone $\beta$ avec la liaison en position 3 du cycle de pyridyle) ou (Y)D-Trp, Y représentant $NO_2$ ou $N^{in}$For ; $R_4$ représente Ser, Orn, AAL ($\beta$-amino-alanine) ou aBu (acide $\gamma$-diaminobutyrique) ; $R_6$ représente D-Trp, $\beta$-D-NAL, (imBzl)D-His ou D-PAL (l'isomère D d'alanine qui a été substituée par le pyridyle sur l'atome de carbone $\beta$) ; $R_7$ représente Nle, Leu, NML(N $CH_3$-L-Leu), Phe, Met, Nva, Tyr, Trp, Cys, PAL ou 4F-D-Phe ; et $R_{10}$ représente Gly-$NH_2$, D-Ala-$NH_2$ ou NH-Y', Y' représentant un alcoyle inférieur, un cycloalcoyle, un fluoro-alcoyle inférieur ou NHCOHN-Q, Q représentant H ou un alcoyle inférieur, ledit procédé comprenant : (a) la formation d'un composé intermédiaire répondant à la formule : $X^1$-$R_1$-(W)-D-Phe-$R_3(X^2)$-$R_4$ $(X^3)$Arg$(X^5)$-$R_6(X^5)$-$R_7(X^6)$-Arg$(X^5)$-Pro-$X^7$, dans laquelle $X^1$ représente un atome d'hydrogène ou un groupe protecteur $\alpha$-amino ; $X^2$ représente un atome d'hydrogène ou un groupe protecteur de l'azote d'indole ; $X^3$ représente un atome d'hydrogène ou un groupe protecteur du groupe hydroxyle alcoolique de Ser ou d'un groupe amino d'une chaîne latérale; $X^5$ et $X^6$ représentent chacun soit un atome d'hydrogène, soit un groupe protecteur de la chaîne latérale respectivement présente ; et $X^7$ est choisi dans le groupe formé de Gly-O-$CH_2$-(support de résine), O-$CH_2$-(support de résine), D-Ala-O-$CH_2$-(support de résine), Gly-NH-(support de résine), D-Ala-NH-(support de résine), Gly-$NH_2$, et des esters, des amides et des hydrazides ; (b) le clivage d'un ou plusieurs des groupes $X^1$ à $X^6$ et/ou le clivage de n'importe quel support de résine compris dans $X^7$ et, si on le souhaite, (c) la transformation d'un peptide résultant en un sel atoxique de celui-ci.

2. Procédé selon la revendication 1, dans lequel W représente 4Cl ou 4F.

3. Procédé selon la revendication 1 ou 2, dans lequel $R_1$ représente $\beta$-D-2NAL et $R_6$ représente D-PAL.

4. Procédé selon la revendication 2, dans lequel $R_6$ représente (imBzl)D-His.

5. Procédé selon l'une quelconque des revendications 1 à 4, dans lequel $R_7$ représente Tyr ou 3PAL.

6. Procédé selon l'une quelconque des revendications 1 à 5, dans lequel $R_3$ représente (Y)D-Trp.

7. Procédé selon la revendication 1 ou 2, dans lequel $R_1$ représente $\beta$-D-NAL et $R_3$ représente D-PAL.

8. Procédé selon la revendication 7, dans lequel $R_6$ représente (imBzl)D-His ou $(6NO_2)$D-Trp et $R_7$ représente Leu.

9. Procédé selon l'une quelconque des revendications 1 à 8, dans lequel $R_4$ représente Ser, X représente Ac ou Acr et $R_{10}$ représente D-Ala-$NH_2$ ou Gly-$NH_2$.

10. Composition pharmaceutique pour réguler la sécrétion de gonadotrophines chez les mammifères, caractérisée en ce qu'elle comprend comme ingrédient actif une quantité efficace d'un peptide selon l'une quelconque des revendications 1 à 9, conjointement avec un diluant ou excipient pharmaceutiquement acceptable.

**Patentansprüche**

**Patentansprüche für folgende Vertragsstaaten: BE, CH, DE, FR, GB, IT, LI, LU, NL, SE.**

1. Peptid oder nicht-toxisches Salz davon, wobei das Peptid die Formel
X-$R_1$-(W) D-Phe-$R_3$-$R_4$-Arg-$R_6$-$R_7$-Arg-Pro-$R_{10}$

12

hat, worin X Wasserstoff oder eine Acylgruppe mit 7 oder weniger Kohlenstoffatomen ist; $R_1$ Dehydro-Pro oder $\beta$-D-NAL (das D-Isomer von Alanin, das durch Naphthyl am $\beta$-Kohlenstoffatom substituiert ist), ist; W Cl, F, $NO_2$, $C^\alpha Me/4Cl$, $Cl_2$ oder Br ist; $R_3$ D-3PAL (das D-Isomer von Alanin, das durch Pyridyl am $\beta$-Kohlenstoffatom mit der Verbindung zu der 3-Position des Pyridylrings substituiert ist) oder (Y)D-Trp ist, wobei Y $NO_2$ oder $N^{in}$For ist; $R_4$ Ser, Orn, AAL ($\beta$-Aminoalanin) oder aBu ($\gamma$-Diaminobuttersäure) ist; $R_6$ D-Trp, $NO_2$-D-Trp, D-Leu, D-Ile, D-Nle, D-Tyr, D-Val, D-Ala, D-Ser(OtBu), $\beta$-D-NAL (imBzl)D-His oder D-PAL (das D-Isomer von Alanin, das durch Pyridyl am $\beta$-Kohlenstoffatom substituiert ist) ist; $R_7$ Nle, Leu, NML($N^\alpha CH_3$-L-Leu), Phe, Met, Nva, Tyr, Trp, Cys, PAL oder 4F-D-Phe ist; und $R_{10}$ Gly-$NH_2$, D-Ala-$NH_2$ oder NH-Y' ist, wobei Y' ein niedriges Alkyl, Cycloalkyl, niedriges Fluoralkyl oder NHCOHN-Q ist, worin Q H oder niedriges Alkyl ist.

2. Peptid nach Anspruch 1, worin W 4Cl oder 4F ist.

3. Peptid nach Anspruch 1 oder Anspruch 2, worin $R_1$ $\beta$-D-2NAL und $R_6$ D-PAL ist.

4. Peptid nach Anspruch 2, worin $R_6$ (imBzl)D-His ist.

5. Peptid nach einem der Ansprüche 1 bis 4, worin $R_7$ Tyr oder 3PAL ist.

6. Peptid nach einem der Ansprüche 1 bis 5, worin $R_3$ (Y)D-Trp ist.

7. Peptid nach Anspruch 1 oder Anspruch 2, worin $R_1$ $\beta$-D-NAL und $R_3$ D-PAL und $R_5$ Arg ist.

8. Peptid nach Anspruch 7, worin $R_6$ (imBzl)D-His oder ($6NO_2$)D-Trp und $R_7$ Leu ist.

9. Peptid nach einem der Ansprüche 1 bis 7, worin $R_4$ Ser, X Ac oder Acr und $R_{10}$ D-Ala-$NH_2$ oder Gly-$NH_2$ ist.

10. Pharmazeutische Zusammensetzung zur Regulierung der Sekretion von Gonadotropinen in Säugetieren, die als einen aktiven Inhaltsstoff eine wirksame Menge eines Peptides nach einem der Ansprüche 1 bis 8, zusammen mit einem pharmazeutisch akzeptierbaren Verdünnungsmittel oder Träger enthält.

**Patentansprüche für folgenden Vertragsstaat: AT**

1. Verfahren zur Herstellung eines Peptides oder nichttoxischen Salzes davon mit der Formel
X-$R_1$-(W) D-Phe-$R_3$-$R_4$-Arg-$R_6$-$R_7$-Arg-Pro-$R_{10}$,
worin X Wasserstoff oder eine Acylgruppe mit 7 oder weniger Kohlenstoffatomen ist; $R_1$ Dehydro-Pro oder $\beta$-D-NAL (das D-Isomer von Alanin, das durch Naphthyl am $\beta$-Kohlenstoffatom substituiert ist) ist; W Cl, F, $NO_2$, $C^\alpha Me/4Cl$, $Cl_2$ oder Br ist; $R_3$ D-3PAL (das D-Isomer von Alanin, das durch Pyridyl am $\beta$-Kohlenstoffatom mit der Verbindung zu der 3-Position des Pyridylrings substituiert ist) oder (Y)D-Trp ist, wobei Y $NO_2$ oder $N^{in}$For ist; $R_4$ Ser, Orn, AAL ($\beta$-Aminoalanin) oder aBu ($\gamma$-Diaminobuttersäure) ist; $R_6$ D-Trp, $\beta$-D-NAL, (imBzl)D-His oder D-PAL (das D-Isomer von Alanin, das durch Pyridyl am $\beta$-Kohlenstoffatom substituiert ist) ist; $R_7$ Nle, Leu, NML($N^\alpha CH_3$-L-Leu), Phe, Met, Nva, Tyr, Trp, Cys, PAL oder 4F-D-Phe ist; und $R_{10}$ Gly-$NH_2$, D-Ala-$NH_2$ oder NH-Y' ist, wobei Y' ein niedriges Alkyl, Cycloalkyl, niedriges Fluoralkyl oder NHCOHN-Q ist, worin Q H oder niedriges Alkyl ist; wobei das Verfahren umfaßt (a) Bilden einer Zwischenverbindung mit der Formel:
$X^1$-$R_1$-(W)D-Phe-$R_3$($X^2$)-$R_4$($X^3$)-Arg($X^5$)-$R_6$($X^5$)-$R_7$($X^6$)-Arg($X^5$)-Pro-$X^7$, worin $X^1$ Wasserstoff oder eine $\alpha$-Aminoschutzgruppe ist; $X^2$ Wasserstoff oder eine Schutzgruppe für den Indolstickstoff ist; $X^3$ Wasserstoff oder eine Schutzgruppe für die alkoholische Hydroxylgruppe von Ser oder für eine Seitenkettenaminogruppe ist; $X^5$ und $X^6$ jeweils entweder Wasserstoff oder eine Schutzgruppe für die entsprechende Seitenkette, die anwesend ist, ist; und $X^7$ ausgewählt ist aus der Gruppe, bestehend aus Gly-O-$CH_2$-(Harzstütze), O-$CH_2$-(Harzstütze), D-Ala-O-$CH_2$-(Harzstütze), Gly-NH-(Harzstütze), D-Ala-NH-(Harzstütze), Gly-$NH_2$ und Estern, Amiden und Hydraziden; (b) Abspalten einer oder mehrerer der Gruppen $X^1$ bis $X^5$ und/oder Abspalten von jeglicher Harzstütze, die in $X^7$ eingeschlossen ist und, wenn gewünscht, (c) Überführung des resultierenden Peptids in ein nicht-toxisches Salz davon.

2. Verfahren nach Anspruch 1, worin W 4Cl oder 4F ist.

3. Verfahren nach Anspruch 1 oder Anspruch 2, worin $R_1$ $\beta$-D-2NAL und $R_6$ D-PAL ist.

4. Verfahren nach Anspruch 2, worin $R_6$ (imBzl)D-His ist.

5. Verfahren nach einem der Ansprüche 1 bis 4, worin $R_7$ Tyr oder 3PAL ist.

6. Verfahren nach einem der Ansprüche 1 bis 5, worin $R_3$ (Y)D-Trp ist.

7. Verfahren nach Anspruch 1 oder Anspruch 2, worin $R_1$ $\beta$-D-NAL und $R_3$ D-PAL ist.

8. Verfahren nach Anspruch 7, worin $R_6$ (imBzl)D-His oder (6NO$_2$)D-Trp und $R_7$ Leu ist.

9. Verfahren nach einem der Ansprüche 1 bis 8, worin $R_4$ Ser, X Ac oder Acr und $R_{10}$ D-Ala-NH$_2$ oder Gly-NH$_2$ ist.

10. Pharmazeutische Zusammensetzung zur Regulierung der Sekretion von Gonadotropinen in Säugetieren, die als aktiven Inhaltsstoff eine wirksame Menge eines Peptides, wie in einem der Ansprüche 1 bis 9 definiert, enthält zusammen mit einem pharmazeutisch akzeptierbaren Verdünnungsmittel oder Träger.